# EUROPEAN PATENT SPECIFICATION

(11) **EP 2 448 831 B1**
(45) Date of publication and mention of the grant of the patent: **19.11.2014**
(21) Application number: 10794439.9
(22) Date of filing: 24.06.2010
(51) Int. Cl.: B65B 31/04, B65B 55/10, A61L 2/06, A61L 2/20

(54) **A DEVICE AND A METHOD FOR GASEOUS-FLOW TREATMENT OF PACKAGES**
VORRICHTUNG UND VERFAHREN ZUR GASSTROMBEHANDLUNG VON VERPACKUNGEN
DISPOSITIF ET PROCEDE DE TRAITEMENT D'EMBALLAGES PAR ECOULEMENT GAZEUX

(30) Priority: 03.07.2009 SE 0900906
(43) Date of publication of application: 09.05.2012
(73) Proprietor: Tetra Laval Holdings & Finance S.A., 1009 Pully (CH)
(72) Inventor: APPARUTI, Daniele, 41050 Montale Rangone (IT); LINDBLAD, Ulf, 224 56 Lund (SE); OLSSON, Jenny, 237 33 Bjärred (SE)
(74) Representative: Tetra Pak - Patent Attorneys SE
(86) International application number: PCT/SE2010/000178
(87) International publication number: WO 2011/002381

(56) References cited:
- EP-A1- 1 944 263
- WO-A1-2007/024173
- DE-A1-102006 036 763
- JP-A- 9 058 632
- JP-A- 2004 352 270
- JP-A- 2009 274 741
- US-A- 4 742 667
- US-A1- 2004 208 781

## Description

### Technical Field

The present invention relates to a device and method used in gaseous-flow treatment of packaging containers, more particularly ready-to-fill packaging containers, having an essentially tubular body provided with a shoulders and an opening arrangement at the one end, and being, at the time of treatment, open at the other end.

### Technical Background

The present invention is particularly well-suited for preheating of packaging containers prior to sterilization treatment in a filling station, but may also be used in gassing/sterilization of containers. In the following sterilization should be interpreted in a broad sense to designate the provision of a level of sterilization corresponding to a particular demand. This demand may vary for various packaging containers and various foodstuffs to be contained in the packaging containers ("packages" in the following), and their corresponding shelf life. This variable level of sterilization is commonly referred to as "commercially sterile".

Packages according to the above definition are sterilized with a sterilization agent prior to a subsequent filling, and this process occurs in a filling machine, such that the packages are sterilized and subsequently filled through an open end thereof, while being arranged in an aseptic environment until the open end has been sealed. Before filling any residual sterilizing agent should be vented, so as not to affect the taste or appearance of the product contained in the package.

Prior to sterilization the packages are heated to a temperature above the dew point of the gaseous sterilization agent. If not, gaseous sterilization agent would be prone to condensate onto surfaces of the package, which would jeopardize the success of the venting. This step in the sterilization process is commonly referred to as preheating.

Strategies elating to filling stations are discussed in WO2004/054883 and WO2007/024173.

The present invention relates mainly to the step of preheating the package, yet the device and method may also be used to flush a package with a gas, which also will be discussed. As mentioned above, the main application relates to ready-to-fill packages, having an open and a closed end, wherein the open end is used as an access route for preheating, sterilizing, flushing (or venting) and filling.

Returning to the preheating step, a prior art method includes the use of an offset injection nozzle for injecting hot air into the package via its open end. The injection nozzle is offset relative to a central axis along the length direction of the package in order to create a flow passing all inside surfaces of the package. Another reason for using a directed flow is that it provides a rapid heating of the inside surface of the package. One alternative would be to use bulk heating of an entire section of the filling machine, and thus of the packages occupying that section of the filling machine. This approach would, however, be significantly slower with a residence time for the packages in the heated section in the order of several minutes, and possibly less energy efficient too. Using a directed flow for preheating may reduce the residence time to a few seconds, which is in harmony with the rest of the operations performed in the filling machine.

A problem with the prior art offset nozzle is, however, that the temperature distribution over the inside surface of the container will be heterogeneous, which will be elaborated in the detailed description. During preheating, the entire inside surface of the package should reach a threshold temperature, above the dew point of the sterilization agent. The heterogeneity will thus inevitably result in that some areas will be overly heated in order for other areas to reach the threshold temperature. This may require an excessive use of energy, and also affect the properties of the packaging material in a negative way.

### Summary

The present invention aims at eliminating or at least alleviating the drawbacks found in prior art by the provision of an injection device according to claim 1, and an injection method according to claim 12. Preferred embodiments are defined by the dependent claims.

The present invention also provides a filling machine having the inventive injection device.

### Brief Description of the Drawings

Fig. 1 illustrates the steps involved in a filling machine for filling food product into a package container.
Fig. 2 schematically illustrates a first embodiment of the present invention, (the circumferential slit is not shown)

Fig. 3 illustrates a second embodiment of the present invention (the circumferential slit is not shown).
Fig. 4 illustrates the geometry of a slit defined between an injection device according to one embodiment of the invention and an open end of a package, during use of one embodiment of the invention.
Figs. 5a and b illustrates schematic illustrations of the flow pattern inside two packages of various dimensions.
Fig. 6 is a cross sectional view of a shield in one embodiment of the invention, indicating various nozzle orientations.

### Detailed Description

Embodiments of the present invention will now be described referring to the drawings. Reference numbers of like components in the various drawings follow an easily understandable pattern.

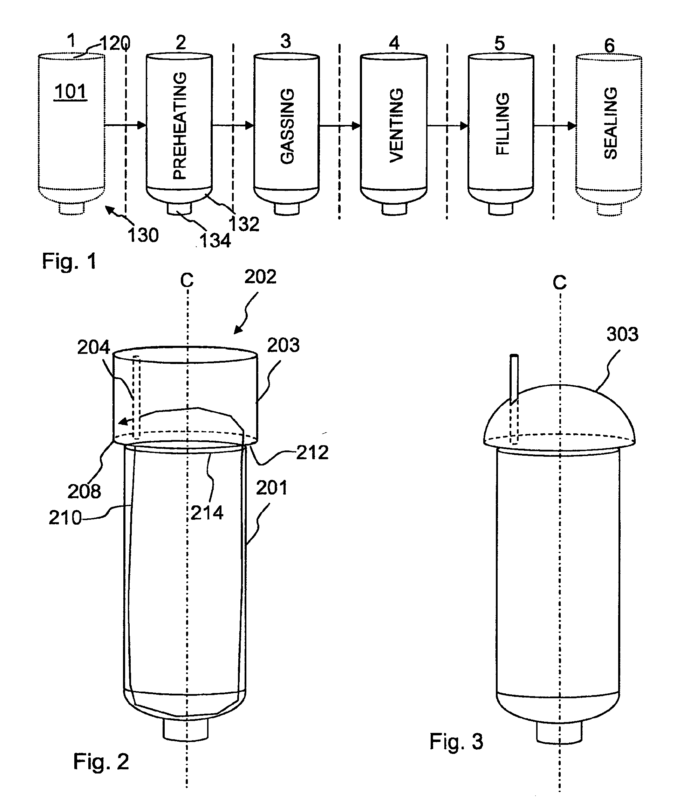

Fig. 1 illustrates the main steps commonly included in a process performed by a filling machine. In a first step 1 the packages 101 are loaded onto a conveyor (not shown), which carries the packages 101, throughout the process. The packages 101 generally have a body of packaging laminate, and an open end 120 and a closed end 130, the latter being provided with shoulders 132 and an opening device 134. In a second step 2 the packages 101 are preheated to a temperature of about 70 °C. The preheating is performed so as to avoid condensation of sterilization agent, which is applied to the packages 101 in a third step 3. After sterilization the packages 101 are vented in a fourth step 4, whereby remains of the sterilization agent are removed. The fifth step 5 involves filling the packages 101, after which the open end of each package 101 is sealed. The atmosphere in filling machine is HEPA-filtered air, and starting from the sterilization step 3 it will also be sterile. As is illustrated in Fig. 1 the different steps are performed in individual sections of the filling machine. The packages 101 are transported sequentially from one step to another by means of a transportation arrangement. The transportation direction may be referred to as the machine direction. In a practical case one there is not a hermetical seal between adjacent sections. Further, in practical cases normally several packages 101 are subjected to the treatment in a step simultaneously, and are indexed forward in the process, e.g., two at a time, and subjected to the treatment of one step while being in a static position. The present invention may however also be used in a configuration where packages 101 are continuously transported in the machine direction, linearly or along a curved path, such as in a rotational filler.

The present inventors have realized that the entrainment of surrounding air is an important cause for the heterogeneous temperature distribution, and Fig. 2 illustrates an injection device 202 according to a first embodiment of the present invention. The device 202 is arranged in register with a package 201 to be preheated, and comprises a shield 203 and an injection nozzle 204. The shield 203 and the nozzle 204 are manufactured from a material able to withstand the heat and forces involved in the injection process, as well as to withstand the sterilization agent(s) and cleaning agent(s) used. Also the material should be easy to clean and to sterilize itself, and examples of such materials include, but are not limited to: PEEK (polyetheretherketone) and stainless steel. The choice of material is also limited to materials approved for food contact in prevailing regulations. Obviously the shield 203 and the nozzle 204 do not have to be manufactured from the same material. Hydrogen peroxide is an example of a commonly used sterilization agent.

The nozzle 204 projects sealingly through the shield 203, so that the only major escape route from the shield 203 is through its opening 206, defined by the lower rim 208 ("lower" referring to the orientation illustrated in Fig. 2). In the illustrated example the nozzle 204 is arranged parallel to the central axis C of the shield 203, in a position offset from said central axis C. In use, this will result in that the flow, illustrated by the curved arrow 210 of hot, sterile air is directed along one side of the package 201, is reversed in the lower part of the package 201, and follows the opposite side as it leaves the package 201. The momentum of the gas flow will force it towards the walls of the shield 203 and combined with the pressure buildup caused by the slit 212 the hot air will be distributed in the confined volume of the package 201 and the shield 203 to such an extent that the temperature distribution will be less heterogeneous. The inventive constructions results in the nozzle shield 203 and the package 201 defining a chamber, which to some extent is isolated from the surroundings. Therefore, the flow entrainment of the jet will be supported, or fed, by heated air from the package rather than from the surroundings, which will reduce the temperature difference between the air injected by the nozzle and the air leaving the package via the slit 212 significantly compared to prior art. This latter effect of self-entrainment is presently also considered as being a main reason for the generation of a more narrow temperature distribution. Simulations have shown a significant effect of the shield in regard of the temperature distribution when compared to a setup without a shield. The effect of the more narrow temperature distribution is that the threshold temperature may be safely exceeded throughout the package, without reaching local temperatures above a level being detrimental for the packaging material.

It should be noted that the flow illustrated in the drawings is highly simplified, and only serves the purpose of facilitating the understanding of the invention. However, for the purposes of the present invention it is preferential that the nozzle does not extend beyond the upper rim of the package, since this would require for the nozzle, the package or both to be movable in the vertical direction. Such a movable arrangement would be possible, yet overly complicated. Also, more than one nozzle may be arranged within the same shield.

Fig. 3 illustrates an embodiment essentially identical to the first, but for the shape of the shield 303, which in contrast to the cylindrical shape of the shield 203 is dome shaped. This is to illustrate that there are several possibilities for the shield shape. If the volume contained inside the shield is not too small, the properties of the slit will have a greater impact on the function of the injection system than the actual surface shape of the shield. Yet, it falls within the scope of the present invention, as defined by the claims, to modify the shape of the shield beyond what is described in the drawings. Reference numbers have been omitted since the similarity with Fig. 2 render them obsolete.

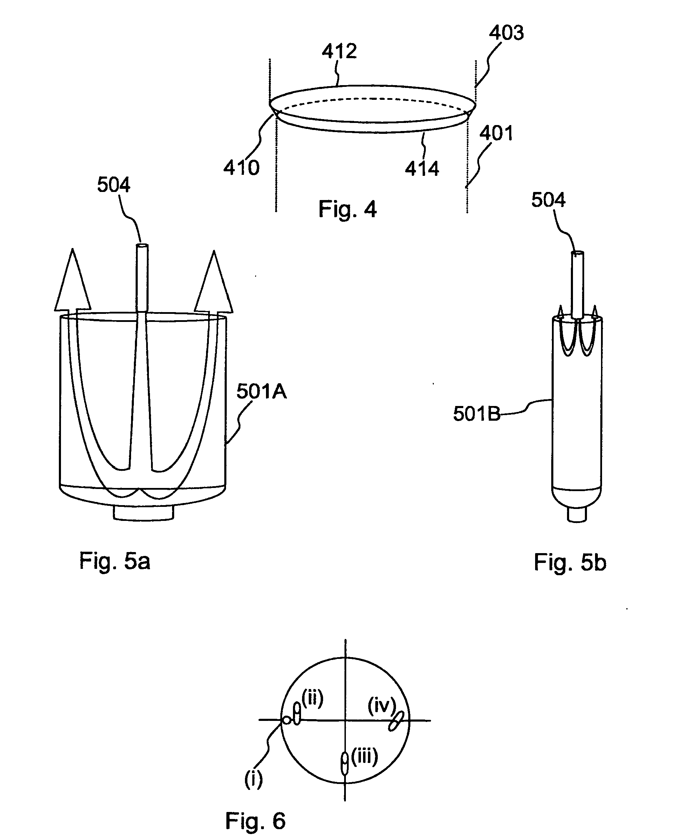

The restriction or slit 412, shown in Fig. 4, which is created by the arrangement of the shield 403 with its rim 412 slightly above the rim 414 of the open end of the package 401 is defined by - assuming that the openings in the shield 403 and package 401 are circular and aligned - the lateral surface area A of the imaginary truncated cone created between them.

For one embodiment the diameter of the shield is 110 mm, the diameter of the package is 80 mm, and the diameter of the nozzle is 11 mm. The flow rate from the nozzle is 60 m³/h, resulting in a flow velocity of approximately 200 m/s.

There will consequently be a rapid, slight pressure build-up in the package, resulting in a low entrainment of surrounding gases. If at all existing, the entrainment is limited to the first short period of time prior to the marginal pressure buildup. The governing effects are thus self-entrainment and pressure buildup, of which the former is clearly dominating.

There may be several beneficial effects from the arrangement of the shield.
- The stream of hot gas or gaseous sterilizing agent will impact on the curved inner surface of the shield, and direct and distribute the stream around the inner perimeter of the shield, and out through the annular slit between the circumference of the packaging container and the shield. This effect may in some case be enhanced by the offset nozzle, which causes the flow to follow the inside of the package container from left to right in Fig. 2.
- Depending on the dimensions of the annular slit it will cause a more or less significant pressure drop. This pressure drop acts to distribute the flow through the slit evenly around its circumference.
- As the flow leaves the slit, it will follow the outer surface of the packaging container to a higher extent than in prior art, which means that the outside of the packaging container will be heated/sterilized to a higher extent.
- The shield will assist in preventing the rapid flow of gases from disturbing the surrounding atmosphere, which may be a beneficial feature in a filling machine.

It should be noted that the drawings by no means illustrate realistic flow patterns. The drawings merely serve the purpose of schematically illustrating some embodiments of the invention.

There are numerous ways of designing alternative embodiments within the scope of the present invention as defined by the claims, some examples are included below:
The shape of the shield may not necessarily be cylindrical, it could for instance be dome shaped, or have another shape deviating from the cylindrical. It could thus also have an inside shape being provided with protrusions or indentations in order to guide the flow further. A reason for deviating from the cylindrical form would be if the package cross section would e.g. be square or rectangular. In such a case the cross section of the shield would correspond to this shape such that a suitable constant slit is created between the rim of the shield and the rim of the package. The basic function of the shield is to capture the return flow from the package and to give the jet the possibility of entraining the preheated return flow. Further, it is beneficial if the flow from the slit is directed downwards such as to avoid disturbing the surrounding flow. These functions are achieved even with the simple geometry presented in the drawings.

In the example illustrated in Fig. 2, the nozzle has a position being offset from the central axis of the shield. It may however have other positions, depending on flow velocities and package size. Some alternatives are illustrated in Fig. 6, where (i) illustrates a nozzle as arranged in the first and second embodiment, and (ii)-(iv) nozzles inclined in various directions relative to the central axis C, creating various flow patterns in the package. If the aspect ratio of the package 501 B, define as the quotient between package height and hydraulic diameter, is too large it will be difficult or impossible to reach the closed end of the package 501 B with a nozzle 504 located in the central axis of the shield, and directed parallel to said central axis. This effect is illustrated in Fig. 5B, and for some commercial packages and flows this may be the case, meaning that this arrangement will be avoided for said type of packages. If, however, the inventive device is to be used for a package 501A having a different aspect ratio, a central arrangement of the nozzle 504 may instead be preferred, as illustrated in Fig. 5A.

In this context it should be mentioned that the central axis of the shield is defined as an axis directed orthogonal to a surface defined by the shield opening. In use the central axis of the shield generally coincides with the central axis of the package. If the shield has a rotational symmetry the central axis generally coincides with the axis of symmetry.

During heating of the package, use is often made of hot, filtered air, which is injected into the system, at a temperature of about 100 °C, with the purpose of heating the package to a temperature exceeding the dew point of the sterilization agent used, without detrimental effects on the packaging laminate. The supply of hot, filtered air to the nozzle is provided by a fan and heater arrangement in a recirculated system.

Examples of other uses for embodiments of inventive device may generally be situations where a package with one open end is to be filled with a gaseous medium. One specific example is when fruit juice is to be filled in a package. Before filling, the sterile air inside the container needs to be replaced by a well defined gas mixture, such as nitrogen or another inert gas, in order to prolong the shelf life of the fruit juice and avoid discolorations etc. Embodiments of the inventive device may then be used for this purpose. Embodiments of the inventive device may also be used in gassing of packages, for sterilization purposes.

According to the present invention, there is also arranged a small, essentially circumferential slit in the uppermost lateral portion of the shield. This slit will not affect the function of the shield significantly, yet it facilitates automatic cleaning of that part of the filling machine, and is as such highly beneficial. The cleaning is simplified since the sharp corner between the shield and overlying structure that would otherwise be present would be more difficult, or even impossible, to clean automatically. A support structure will fix the shield to the overlying structure.

If an embodiment of the inventive device is to be used for injection of sterilization agent, or for ventilation of packages, the flow rate of the injection nozzle(s) may have to be adjusted. It is considered to fall within the competence of the skilled person to make these adjustments based on the teachings of the present invention.

The present invention may be applied in a filling or packaging machine, further details of which are described in a number of copending Swedish patent applications, filed by the same applicant on the same day as the present application. To this end further details of:
A device and method for maintaining asepticity is disclosed in "A device and a method for maintaining a gas flow barrier between two interconnected volumes" (SE-0900911-9), of which an alternative device and method is disclosed in "A device and a method for maintaining a gas flow barrier between two volumes of a channel" (SE-0900913-5).
A method for obtaining an optimized concentration of sterilization agent in a sterilization zone is disclosed in the application with the title "A device and a method for sterilizing packages" (SE-0900907-7).
A system for ensuring that entrainment air is present for the jet flows of the filling zone and venting zone is disclosed in the application with the title "A system for treating packaging containers" (SE-0900912-7).
A device for providing cleaned air, which may be used for the as a source of entrainment air to jets in the venting zone and filling zone and surplus air in the filling zone, is disclosed in the application with the title "A device for cleaned air provision" (SE-0900908-5).

Some various aspect of the filling or packaging machine are disclosed in the applications titled "Packaging machine and packaging method I" (SE-0900909-3) and "Packaging machine and packaging method II" (SE-0900910-1), respectively. A system for supplying entrainment air to jet air flows in the machine are disclosed in the application with the title "A system for treating packaging containers" (SE-0900912-7).

## Claims

1. An injection system for injecting gaseous media into the open end of a packaging container, comprising
a nozzle (204;304;404;504) directing the gaseous media into the container (201; 301; 401;501),
**characterized in that** it further comprises a shield (203; 303; 403; 503) arranged around the nozzle (204-504), said shield having
a shield opening facing the opening of the packaging container, a cross sectional shape at the shield opening with dimensions being equal to or exceeding the corresponding dimensions of the of the packaging container at its opening facing the shield such that a slit (212; 312; 412; 512) may be created between the perimeter of said openings, wherein the shield has a small, essentially circumferential slit in the end opposed to the shield opening, in an uppermost lateral portion of the shield.

2. The injection system of claim 1, wherein the nozzle is arranged at a distance from the central axis of the shield.

3. The injection system of claim 1, wherein the nozzle is arranged coinciding with the central axis of the shield.

4. The injection system of any preceding claim, wherein the nozzle is arranged at an angle relative to the central axis of the shield.

5. The injection system of any of claims 1-3, wherein the nozzle is arranged parallel to the central axis of the shield.

6. The injection system of any preceding claim, wherein several nozzles are arranged within the shield.

7. The injection system of any preceding claim, wherein the gaseous media comprises hot air.

8. The injection system of any preceding claim, wherein a flow rate of the gaseous media is 60 m³/h.

9. The injection system of any preceding claim, wherein a flow is arranged on the radially outer side of the packaging container.

10. The injection system of claim 1, wherein the cross sectional shape is circular.

11. The injection system of claim 1, wherein the cross sectional shape is rectangular.

12. A method for injecting gaseous media into the open end of a packaging container, comprising the steps of:
arranging a package container having a closed end and an open end with the open end facing an injection nozzle (204;304;404;504),
wherein a shield (203; 303; 403; 503) is arranged around the nozzle (204-504), said shield having a diameter which is equal to or exceeds the diameter of the packaging container and a shield opening facing the opening of the packaging container, such that a slit (212; 312; 412; 512) may be created between the perimeter of said openings, and providing a small, essentially circumferential slit in the end of the shield opposed to the shield opening, in an uppermost lateral portion of the shield, followed by the step of
injecting gaseous media into the packaging container.

## Patentansprüche

1. Einspritzsystem zum Einspritzen von gasförmigem Medien in das offene Ende eines Verpackungsbehälters, umfassend.
eine Düse (204; 304; 404; 504), die das gasförmige Medium in den Behälter (201; 301; 401; 501) leitet,
**dadurch gekennzeichnet, dass** es ferner eine Abschirmung (203; 303; 403; 503) umfasst, die um die Düse (204 bis 504) angeordnet ist, wobei die Abschirmung eine Abschirmungsöffnung aufweist, die zu der Öffnung des Verpackungsbehälters weist, eine Querschnittsform an der Abschirmungsöffnung mit Abmessungen, die den entsprechenden Abmessungen des Verpackungsbehälters an seiner Öffnung entsprechen, die zu der Abschirmung weist, oder diese überschreiten, sodass ein Schlitz (212; 312; 412; 512) zwischen dem Umfang der Öffnungen erzeugt werden kann, wobei die Abschirmung einen kleinen, im Wesentlichen umlaufenden Schlitz in dem Ende gegenüber der Abschirmungsöffnung in einem obersten seitlichen Teil der Abschirmung aufweist.

2. Einspritzsystem nach Anspruch 1, wobei die Düse in einem Abstand von der Mittelachse der Abschirmung angeordnet ist.

3. Einspritzsystem nach Anspruch 1, wobei die Düse mit der Mittelachse der Abschirmung übereinstimmend angeordnet ist.

4. Einspritzsystem nach einem der vorherigen Ansprüche, wobei die Düse in einem Winkel zu der Mittelachse der Abschirmung angeordnet ist.

5. Einspritzsystem nach einem der Ansprüche 1 bis 3, wobei die Düse parallel zu der Mittelachse der Abschirmung angeordnet ist.

6. Einspritzsystem nach einem der vorhergehenden Ansprüche, wobei mehrere Düsen innerhalb der Abschirmung angeordnet sind.

7. Einspritzsystem nach einem der vorhergehenden Ansprüche, wobei das gasförmige Medium heiße Luft umfasst.

8. Einspritzsystem nach einem der vorhergehenden Ansprüche, wobei eine Strömungsrate des gasförmigen Mediums 60 m³/h beträgt.

9. Einspritzsystem nach einem der vorhergehenden Ansprüche, wobei eine Strömung auf der radialen Außenseite des Verpackungsbehälters angeordnet ist.

10. Einspritzsystem nach Anspruch 1, wobei die Querschnittsform kreisförmig ist.

11. Einspritzsystem nach Anspruch 1, wobei die Querschnittsform rechteckig ist.

12. Verfahren zum Einspritzen von gasförmigem Medien in das offene Ende eines Verpackungsbehälters, umfassend die Schritte:
Anordnen eines Verpackungsbehälters mit einem geschlossenen Ende und einem offenen Ende mit dem offenen Ende zu einer Einspritzdüse (204; 304; 404; 504) weisend,
wobei eine Abschirmung (203; 303; 403; 503) um die Düse (204 bis 504) angeordnet ist, wobei die Abschirmung einen Durchmesser aufweist, der gleich oder größer als der Durchmesser des Verpackungsbehälters ist, und eine Verpackungsöffnung zu der Öffnung des Verpackungsbehälters weist, so dass ein Schlitz (212; 312; 412; 512) zwischen dem Umfang der Öffnungen ausgebildet werden kann, und ein kleiner, im Wesentlichen umlaufender Schlitz im Ende der Abschirmung gegenüber der Abschirmungsöffnung in einem obersten seitlichen Teil der Abschirmung bereitgestellt wird, wonach der Schritt des Einspritzens von gasförmigen Medien in den Verpackungsbehälter erfolgt.

## Revendications

1. Système d'injection pour injecter un milieu gazeux dans l'extrémité ouverte d'un récipient d'emballage, comprenant :
une buse (204 ; 304 ; 404 ; 504) dirigeant le milieu gazeux dans le récipient (201 ; 301 ; 401 ; 501),
**caractérisé en ce qu'**il comprend en outre un écran (203 ; 303 ; 403 ; 503) agencé autour de la buse (204-504), ledit écran ayant
une ouverture d'écran faisant face à l'ouverture du récipient d'emballage, une forme en section transversale au niveau de l'ouverture d'écran ayant des dimensions égales ou supérieures aux dimensions correspondantes du récipient d'emballage au niveau de son ouverture tournée vers l'écran, de telle sorte qu'une fente (212 ; 312 ; 412 ; 512) puisse être créée entre le périmètre desdits ouvertures, l'écran ayant une petite fente essentiellement circonférentielle dans l'extrémité opposée à l'ouverture d'écran, dans une portion latérale la plus supérieure de l'écran.

2. Système d'injection selon la revendication 1, dans lequel la buse est agencée à distance de l'axe central de l'écran.

3. Système d'injection selon la revendication 1, dans lequel la buse est agencée de manière à coïncider avec l'axe central de l'écran.

4. Système d'injection selon l'une quelconque des revendications précédentes, dans lequel la buse est agencée suivant un certain angle par rapport à l'axe central de l'écran.

5. Système d'injection selon l'une quelconque des revendications 1 à 3, dans lequel la buse est agencée parallèlement à l'axe central de l'écran.

6. Système d'injection selon l'une quelconque des revendications précédentes, dans lequel plusieurs buses sont agencées à l'intérieur de l'écran.

7. Système d'injection selon l'une quelconque des revendications précédentes, dans lequel le milieu gazeux comprend de l'air chaud.

8. Système d'injection selon l'une quelconque des revendications précédentes, dans lequel un débit du milieu gazeux est de 60 m³/h.

9. Système d'injection selon l'une quelconque des revendications précédentes, dans lequel un écoulement est prévu sur le côté radialement externe du récipient d'emballage.

10. Système d'injection selon la revendication 1, dans lequel la forme en section transversale est circulaire.

11. Système d'injection selon la revendication 1, dans lequel la forme en section transversale est rectangulaire.

12. Procédé pour injecter un milieu gazeux dans l'extrémité ouverte d'un récipient d'emballage, comprenant les étapes suivantes :
disposer un récipient d'emballage ayant une extrémité fermée et une extrémité ouverte avec l'extrémité ouverte tournée vers une buse d'injection (204 ; 304 ; 404 ; 504),
un écran (203 ; 303 ; 403 ; 503) étant agencé autour de la buse (204-504), ledit écran ayant un diamètre qui est égal ou supérieur au diamètre du récipient d'emballage et une ouverture d'écran tournée vers l'ouverture du récipient d'emballage,
de telle sorte qu'une fente (212 ; 312 ; 412 ; 512) puisse être créée entre le périmètre desdites ouvertures, et prévoir une petite fente essentiellement circonférentielle dans l'extrémité de l'écran opposée à l'ouverture d'écran, dans une portion latérale la plus supérieure de l'écran, suivies par l'étape consistant à
injecter un milieu gazeux dans le récipient d'emballage.
